# EUROPÄISCHE PATENTSCHRIFT

(11) **EP 1 927 326 B1**
(45) Veröffentlichungstag und Bekanntmachung des Hinweises auf die Patenterteilung: **28.10.2009**
(21) Anmeldenummer: 06024575.0
(22) Anmeldetag: 28.11.2006
(51) Int. Cl.: A61C 13/00

(54) **Verfahren zur Herstellung einer zahnärztlichen Restauration**
Method of manufacturing a dental restoration
Procédé de fabrication d'une restauration dentaire

(43) Veröffentlichungstag der Anmeldung: 04.06.2008
(73) Patentinhaber: DeguDent GmbH, 63457 Hanau (DE)
(72) Erfinder: Ertl, Thomas, Dr., 63303 Dreieich (DE)
(74) Vertreter: Stoffregen, Hans-Herbert

(56) Entgegenhaltungen:
- EP-A- 1 710 536
- WO-A-2005/058183
- DE-A1- 19 749 107
- US-A1- 2003 219 148
- US-B2- 7 065 243

## Beschreibung

Die Erfindung bezieht sich auf ein Verfahren zur Herstellung einer zahnärztlichen Restauration für zumindest einen Abschnitt eines Zahnbogens auf der Basis von digitalen Gesamtdaten, die aus in Beziehung gesetzten Einzeldaten berechnet werden, denen intraoral durchgeführte dreidimensionale optische Einzelmessungen zumindest eines den Abschnitt enthaltenden Bereichs des Zahnbogens zu Grunde gelegt werden, wobei der zumindest eine Bereich des Zahnbogens zusammen mit einer Referenzierung, die dreidimensionale Informationen zur Verfügung stellt, gemessen wird und aus den Einzeldaten unter Berücksichtigung der Referenzierung die Gesamtdaten berechnet werden.

Um Zahnersatz herzustellen ist es bekannt, von einem Bereich eines Zahnbogens, in den eine zahnärztliche Restauration eingebracht werden soll, ein Duplikat herzustellen. Sodann werden von Abschnitten des Duplikats Einzelscans angefertigt, die sodann zur Berechnung von digitalen Daten räumlich einander zugeordnet werden. Aus den entsprechenden Daten wird z. B. unter Einbeziehung von in einer Bibliothek abgelegten Gestaltungen der Restauration im CAD/CAM-Verfahren die Restauration durch Bearbeiten wie Fräsen eines vorzugsweise aus Keramik bestehenden Rohlings hergestellt.

Um bei der Berechnung der für die Herstellung der zahnärztlichen Restauration erforderlichen Daten messbedingte Fehler zu minimieren, ist es nach der WO-A-2005/058183 bekannt, die gemessenen einzelnen Duplikatabschnitte unter Zugrundelegung einer Referenzierung räumlich einander zuzuordnen.

Auch die DE-B-10 2004 051 165 legt ein abzuscannendes Modell der Herstellung eines Zahnersatzes zu Grunde, wobei gescannte Segmente des Modells mittels einer Referenzierung räumlich zueinander ausgerichtet werden.

Aus der US-A-2003/0219148, auf der die zweiteilige Form des Anspruchs 1 basiert, ist ein Verfahren zur Generierung eines dreidimensionalen Modells eines Zahnbogens auf der Basis von digitalen Daten zu entnehmen, die aus in Beziehung gesetzten Einzeldaten ermittelt werden, die ihrerseits durch dreidimensionale optische Einzelmessungen gewonnen werden. Um sich überlappende Einzelmessungen durch Stitchen zusammenfügen zu können, werden dreidimensionale Referenzierungen mitgescannt, die teilweise zu scannende Bereiche abdecken können.

Gegenstand der EP-A-1 710 536 ist ein Verfahren zur dreidimensionalen Formerfassung insbesondere eines zahntechnischen Modells. Bereiche des Modells werden zusammen mit einem Referenzobjekt optisch gemessen, wobei aus der Abweichung zwischen Ist- und Realwerten des Referenzobjektes Korrekturwerte ermittelt werden.

Bei einem Verfahren zur Ermittlung der Form eines mit einer zahnärztlichen Restauration zu versehenen Duplikats eines Restzahnbereichs nach der WO-A-2005/058183 werden Duplikatsabschnitte in ihrer räumlichen Zuordnung zueinander auf der Basis einer in einem Rechner abgelegten Referenzierung referenziert.

Ein Standard-Maßstab zur Verwendung mit einer fotogrammetrischen Meßeinrichtung wird in der DE-A-197 49 107 beschrieben.

Der US-B-7,065,243 ist ein Verfahren zur Erstellung eines Zahnmodells zu entnehmen. Dabei wird durch intraorales Messen eines oder mehrerer Zähne bei gleichzeitiger Gegenwart einer Referenz durch diese die Position einer Kamera bestimmt, mittels der der bzw. die Zähne gemessen werden. Nach Kenntnis der Kameraposition in Bezug auf den bzw. die Zähne wird sodann aus den Messungen ein 3D-Modell erzeugt, das für die Herstellung von Zahnersatz benötigt wird. Ein entsprechendes Verfahren ist aufwändig und zeigt zudem den Nachteil, dass die als quaderförmiger offener Kasten ausgebildete Referenz den Zahn bzw. die Zähne abschattet.

Beim intraoralen Messen zumindest eines Abschnitts eines Zahnbogens wird dieser von verschiedenen Seiten gemessen, wobei der Abstand der Kamera variiert. Die Einzelaufnahmen werden sodann durch Stitchen und Mergen zur Gewinnung dreidimensionaler Daten des Abschnitts des Zahnbogens zusammengefügt, um sodann auf der Basis dieser digital zur Verfügung stehenden Daten den gewünschten Zahnersatz herstellen zu können. Dabei kann es durch kumulative Anhäufung kleiner Abweichungen zu Verzerrungen des 3D-Datensatzes kommen, die die zulässigen Toleranzgrenzen überschreiten.

Der vorliegenden Erfindung liegt die Aufgabe zu Grunde, ein Verfahren der eingangs genannten Art so weiterzubilden, dass die aus Einzelmessungen gewonnenen Gesamtdaten den gemessenen Abschnitt des Zahnbogens bzw. den Zahnbogen insgesamt unverzerrt darstellen, ohne dass bei der Messung des Abschnitts des Zahnbogens Abschattungen zu Messverfälschungen führen können.

Zur Lösung der Aufgabe, sieht die Erfindung im Wesentlichen folgende Verfahrensschritte vor:
a) Durchführen erster intraoraler dreidimensionaler optischer Messungen von dem zumindest einen Bereich des Zahnbogens ohne Vorhandensein der Referenzierung zur Ermittlung erster Einzeldaten,
b) Positionieren der Referenzierung (36, 38) zu dem Bereich,
c) Durchrühren zweiter intraoraler dreidimensionaler optischer Messungen von dem Bereich des Zahnbogens oder einem weiteren (zweiten) Bereich, der den Bereich des Zahnbogens umfasst, zur Ermittlung zweiter Einzeldaten und
d) Verknüpfen der ersten Einzeldaten unter Einbeziehung der die Referenzierung berücksichtigenden zweiten Einzeldaten zum Berechnen der Gesamtdaten unter Berücksichtigung von Korrekturdaten aus der Differenz zwischen gemessener und realer Geometrie der Referenzierung
   oder
e) Verwenden eines zumindest teilweise transparenten Plattenelements mit in zumindest zwei Ebenen verlaufenden Markierungen als die Referenzierung, Messen des zumindest einen Bereichs des Zahnbogens zusammen mit der Referenzierung und Berechnen der Gesamtdaten.

Erfindungsgemäß ist vorgesehen, dass zunächst erste dreidimensionale optische Messungen von dem zumindest einen Bereich des Zahnbogens ohne Vorhandensein der Referenzierung zur Ermittlung erster Einzeldaten erfolgen, sodann die Referenzierung zu dem Bereich positioniert wird und schließlich zweite dreidimensionale Messungen zur Ermittlung zweiter Einzeldaten von einem zweiten Bereich des Zahnbogens erfolgen, der den zumindest ersten Bereich sowie die Referenzierung umfasst. Anschließend erfolgt ein Verknüpfen der ersten Einzeldaten unter Einbeziehung der die Referenzierung umfassenden zweiten Einzeldaten zum Berechnen der Gesamtdaten, wobei Korrekturdaten berücksichtigt werden, die sich aus der Differenz zwischen gemessener und realer Geometrie der Referenzierung ergeben. Dabei kann die Messabfolge auch umgekehrt durchgeführt werden, d. h. zunächst wird Zahnbogen bzw. ein Bereich dieses zusammen mit der Referenzierung gemessen und sodann ohne diese. Die Erfindung wird auch dann nicht verlassen, wenn der Zahnbogen bzw. der relevante Bereich dieses zusammen mit der Referenzierung ohne Durchführung weiterer Messungen gemessen wird. In diesem Fall darf jedoch die Referenzierung den Zahnbogen bzw. dessen Bereich nicht in dem Gebiet abschatten, in dem die zahntechnische Restauration eingebracht werden soll.

Unter Abschnitt eines Zahnbogens ist dabei ein Zahn, eine Gruppe von Zähnen oder sogar der ganze Zahnbogen zu verstehen, der vollbezahnt, teilbezahnt oder auch zahnlos sein kann.

Erfindungsgemäß wird die Referenzierung bekannter dreidimensionaler Geometrie entweder zusammen mit dem relevanten Bereich des Zahnbogens optisch gemessen oder im Falle einer teilweisen Abschattung wird ohne die Referenzierung der relevante bereich des Zahnbogens ermittelt, um sodann durch Stitchen und Mergen der Einzelmessungen unter Berücksichtigung der Referenzierung dreidimensionale Daten des relevanten Bereich des Zahnbogens zu erhalten, auf deren Basis der Zahnersatz hergestellt wird. Die die Referenzierung aufweisenden Einzelmessungen werden zusammengefügt, wobei aufgrund der Kenntnis der Geometrie der Referenzierung eine Korrektur der zusammengesetzten Messungen erfolgt.

Es werden in gewohnter Weise die Einzelmessungen durch übliche Rechenverfahren unter Berücksichtigung der sich überlappenden Bereiche zur Gewinnung einer 3D-Darstellung des Zahnbogens bzw. des relevanten Bereichs zusammengefügt, um anschließend eine Korrektur der Ist-Referenzierung auf die bekannte Referenzierung durchzuführen, um eine verzerrungsfreie 3D-Darstellung zu erhalten, von der die benötigten digitalen Daten zur Herstellung des Zahnersatzes abgeleitet werden.

Werden Messungen mit und ohne Referenzierung durchgeführt, so werden zum einen die Einzelmessungen ohne Referenzierung und zum anderen die Einzelmessungen mit Referenzierung zur Herstellung jeweils eines dreidimensionalen Bildes zusammengefügt und die erhaltenen dreidimensionalen Bilder durch Auswertung der Unterschiede zwischen Abbildung der Referenzierung und ihrer bekannten tatsächlichen Geometrie korrigiert, d. h. die gemessene Referenzierung, also die Ist-Referenzierung wird in die bekannte tatsächliche oder reale Geometrie der Referenzierung korrigiert. Somit erhält man einen dreidimensionalen Datensatz, ohne dass in Bezug auf den gemessenen Bereich des Zahnbogens Verzerrungen vorliegen. Mit anderen Worten werden aus der Abweichung der gemessenen Daten des Referenzobjekts von seiner tatsächlichen Geometrie Korrekturdaten berechnet. Diese Korrekturdaten werden auf die verzerrten Messdaten angewandt, um den Messfehler zu korrigieren.

Als Referenzierung kann ein Objekt verwendet werden, das eine Plattengeometrie aufweist, wobei das Objekt dreidimensionale Erhebungen und /oder Vertiefungen wie Pyramiden oder Pyramidenstümpfe aufweist, um eine eindeutige dreidimensionale Zuordnung der Einzelmessung zu ermöglichen. Insbesondere kann als Objekt ein zumindest teilweise transparentes Plattenelement mit in zumindest zwei Ebenen vorhandenen vorzugsweise rasterförmigen und versetzt zueinander verlaufenden Markierungen verwendet werden, wodurch Rückschlüsse auf die Kameraposition möglich sind. Je nach Abstand der Ebenen zueinander und Versatz der Markierungen gegeneinander ergeben sich von der Position der Kamera abhängige Abbildungen, die aus der Kenntnis der Referenzierung passgenau zusammengefügt werden, so dass eine Verzerrung des aus den Einzelmessungen ermittelten relevanten Bereichs des Zahnbogens dem Grunde nach ausgeschlossen ist.

Losgelöst hiervon sollte die Referenzierung aus einem Material niedrigen Ausdehnungskoeffizientens und hohen E-Moduls bestehen wie Keramik bzw. Glaskeramik, um temperaturbedingte oder durch Krafteinwirkungen bedingte Fehler zu minimieren.

Weitere Einzelheiten, Vorteile und Merkmale der Erfindung ergeben sich nicht nur aus den Ansprüchen, den diesen zu entnehmenden Merkmalen - für sich und/oder in Kombination -, sondern auch aus der nachfolgenden Beschreibung von der Zeichnung zu entnehmenden bevorzugten Ausführungsbeispielen.

Es zeigen:
- Fig. 1: eine Prinzipdarstellung zum Messen eines dentalen Bereichs mit und ohne Referenzierung,
- Fig. 2: eine Prinzipdarstellung eines Unterkiefers,
- Fig. 3: Einzelaufnahmen eines Bereichs des Unterkiefers gemäß Fig. 2 und
- Fig. 4: eine Darstellung des Unterkiefers gemäß Fig. 2 nach Zusammenfügen der Einzelbilder.

Um eine zahntechnische Restauration herzustellen, wird der Bereich eines Zahnbogens, in dem der Zahnersatz eingesetzt werden soll, dreidimensional gemessen, um sodann aus dem so erhaltenen 3D-Bild digitale Daten zu gewinnen, auf deren Grundlage der Zahnersatz im CAD/CAM-Verfahren hergestellt wird. Da der entsprechende Bereich bzw. gegebenenfalls der gesamte Zahnbogen allumfassend und ohne Abschattungen mit einer Aufnahme nicht erfasst werden kann, ist es üblich, mehrere Einzelbilder - nachstehend auch als Einzelscans bezeichnet - aufzunehmen, die sodann durch bekannte Techniken wie Registrieren, Stitchen und Mergen zusammengefügt werden, wie dies z. B. der DE-A-10 2005 011 066 zu entnehmen ist. Dabei zeigt sich nach dem Stand der Technik der Nachteil, dass durch kumulative Anhäufung kleiner Abweichungen beim Stitchen und Mergen der Einzelscans der zur Herstellung des Zahnersatzes benötigte 3D-Datensatz Verzerrungen beinhaltet, die zulässige Toleranzgrenzen überschreiten. Um diese Verzerrung zu eliminieren, wird erfindungsgemäß der relevante Bereich zusammen mit einer Referenzierung gemessen, durch die es ermöglicht wird, die einzelnen Scans objektgenau zusammenzufügen, so dass entsprechende Verzerrungen unterbleiben.

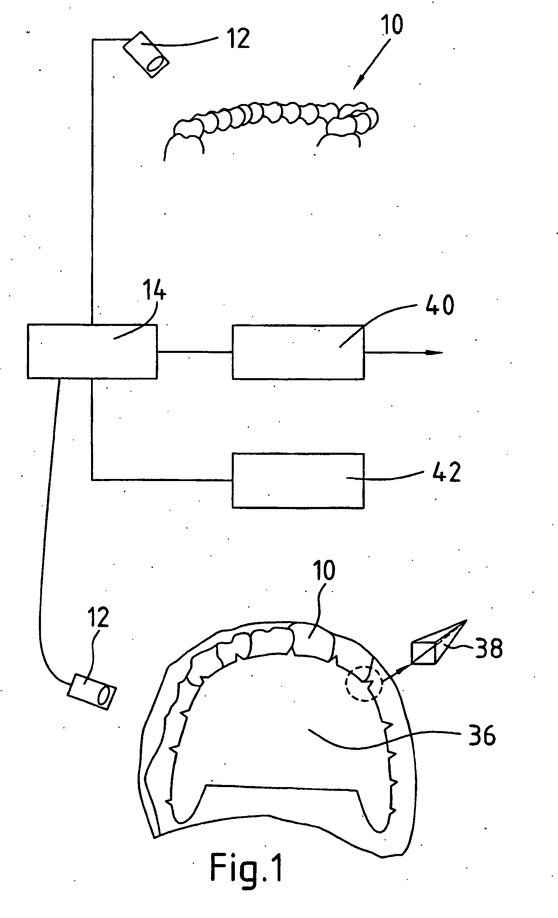

In Fig. 1 ist rein prinzipiell ein Unterkiefer 10 dargestellt, von dem ein Abschnitt optisch dreidimensional gemessen und sodann aus den Einzelmessungen ein 3D-Datensatz gewonnen wird, der zur Herstellung des Zahnersatzes erforderlich ist. Hierzu wird auf den Unterkiefer 10 eine Kamera 12 in verschiedenen Positionen ausgerichtet, um den relevanten Bereich von verschiedenen Positionen aus aufzunehmen. Da entsprechende Messungen manuell erfolgen, verändert sich folglich der Abstand zu dem Unterkiefer 10, so dass die Einzelbilder gestitcht und gemergt werden müssen, um die 3D-Darstellung des relevanten Bereichs des Unterkiefers zu erzielen. Hierzu werden die von der Kamera 12 aufgenommenen Bilder einer Bildverarbeitung mit Rechner 14 zugeführt.

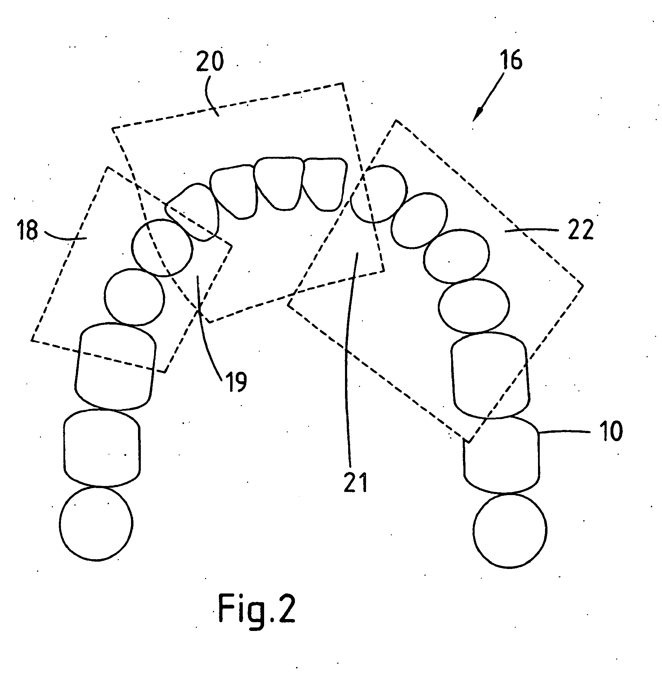

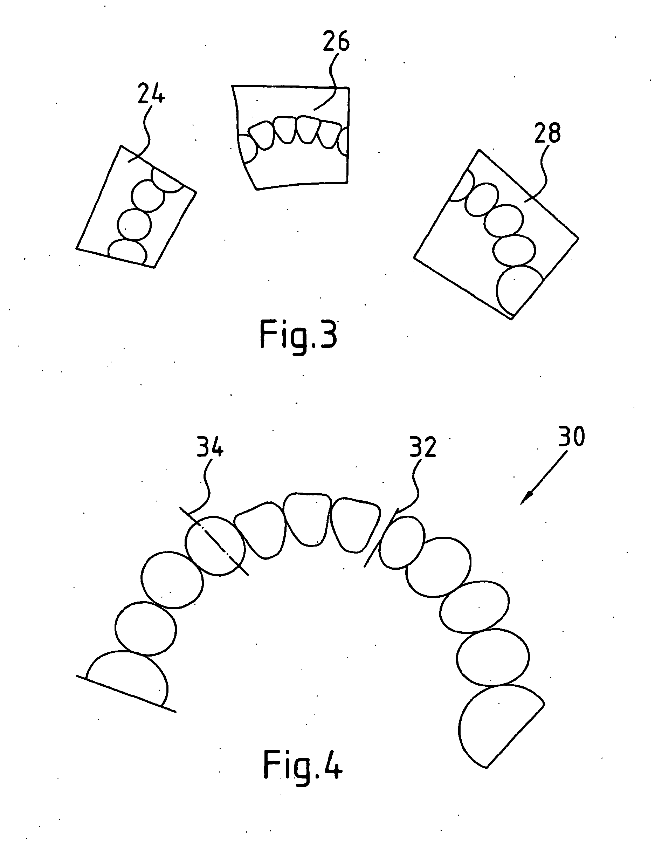

Fig. 2 zeigt den Unterkiefer 10 in Draufsicht, von dem in einem relevanten Bereich, in dem eine zahntechnische Rekonstruktion wie Krone oder Brücke eingesetzt werden soll, unter verschiedenen Winkeln dreidimensionale Bilder aufgenommen werden sollen. Dabei müssen sich die Bilder überlappen, um aus den Einzelbildern (Einzelscans) ein Gesamtscan, also ein 3D-Bild des gesamten relevanten Bereichs herstellen zu können. Rein beispielhaft sind in der Fig. 2 drei Bereiche 18, 20, 22 eingezeichnet, von denen sogenannte Einzelscans angefertigt werden. Da die Kamera 12 die Bereiche 18, 20, 22 in unterschiedlichen Abständen bzw. unter voneinander abweichenden Winkeln erfasst, sind die Bilder der entsprechenden Bereiche weder in der Größe der Abbildung noch in Bezug auf die Raumerfassung gleich. Dies soll anhand der Fig. 3 verdeutlicht werden. So sind Aufsichtaufnahmen der Bereiche 18, 20, 22 prinzipiell dargestellt, die in unterschiedlichem Abstand von dem Unterkiefer 10 aufgenommen worden sind, so dass sich verschiedene voneinander abweichende Vergrößerungen ergeben. Die entsprechenden Bilder sind mit den Bezugszeichen 24, 26, 28 gekennzeichnet. Dabei überlappen sich die Bilder 24, 26, 28, wie sich aus den sich überlappenden Bereichen 19, 21 ergibt. Aufgrund der Überlappung 19, 21 besteht nunmehr die Möglichkeit, die einzelnen Bilder 24, 26, 28 zusammenzufügen, und zwar durch bekanntes Stitchen und Mergen. Hierbei erfolgen eine eventuell nötige Größenanpassung, eine Zusammenführung der 3D-Daten in ein einheitliches Koordinatensystem, eine Ausrichtung zueinander sowie Verwerfen von 3D-Daten mit bekannt geringerer Genauigkeit, sofern Daten mit höherer Genauigkeit im gleichen Objektbereich zur Verfugung stehen. Hierdurch kann es zu kumulativer Anhäufung von Abweichungen kommen, wie sich aus dem zusammengesetzten Bild 30 gemäß Fig. 4 ergibt. So sind in den Überlappungsbereichen übertrieben dargestellte Verzerrungen 32, 34 aufgetreten, die dazu führen, dass auf der Basis des Bildes 30, aus dem bei der Weiterverarbeitung dreidimensional digitale Daten gewonnen werden, Fehler auftreten, die dazu führen, dass der Zahnersatz die erforderliche Passgenauigkeit nicht aufweist.

Um diese Fehler zu eliminieren, ist erfindungsgemäß vorgesehen, dass der relevante Bereich des Unterkiefers 10 zusammen mit einer Referenzierung 36 optisch gemessen wird, die ihrerseits dreidimensionale Strukturen aufweist, um eine eindeutige Zuordnung der einzelnen Bilder unter Berücksichtigung des aus der dreidimensionalen Struktur der Referenzierung sich ergebenden Position der Kamera 12 zu ermöglichen.

Im Ausführungsbeispiel ist die Referenzierung 36 plattenförmig ausgebildet und wird innerhalb des von dem Unterkiefer 10 gebildeten Zahnbogens gelegt. Peripher ragen dreidimensionale im Ausführungsbeispiel pyramidenartige Vorsprünge 38 ab, die die erforderliche Zuordnung der Einzelbilder ermöglichen. Die Einzelbilder oder Einzelscans werden gleichfalls der Bildverarbeitung und dem Rechner 14 zugeführt, so dass die sich aus der Messung des Unterkiefers 10 ohne Referenzierung ermittelte 3D-Darstellung des relevanten Bereichs mit den Bildern des Unterkiefers 10 korreliert und korrigiert werden, bei der die Referenzierung 36 eingesetzt ist. Dabei muss darauf geachtet werden, dass bei den Einzelaufnahmen des Unterkiefers 10 zusammen mit der Referenzierung 36 eine Positionsänderung der Referenzierung 36 zu dem Unterkiefer 10 unterbleibt.

Die Bilder der Unterkiefer 10 aus den Aufnahmen mit und ohne Referenzierung werden durch geeignete Software in Übereinstimmung gebracht, um sodann eine Korrektur derart vorzunehmen, dass die den 3D-Bildern zu entnehmende Referenzierung mit der tatsächlichen Geometrie der Referenzierung 36 übereinstimmt.

Bei der Referenzierung 36 handelt es sich insbesondere um ein plattenförmiges Element, das aus einem Material niedrigen Ausdehnungskoeffizientens und eines hohen E-Moduls besteht. Bevorzugte Materialien sind Keramik wie Glaskeramik.

Die dreidimensionale Strukturierung, die in der Zeichnung beispielhaft als Pyramide dargestellt ist, geht von der Umfangsrandfläche des Referenzierungselementes 36 aus und ermöglicht somit, Verzerrungen in allen 6 Freiheitsgraden zu eliminieren.

Ist anhand des Ausführungsbeispiels erläutert worden, dass das verzerrungsfreie Zusammenfügen der Einzelaufnahmen durch getrennte Aufnahmen des Unterkiefers 10 mit und ohne Referenzierung 36 erfolgt, so besteht auch die Möglichkeit, aus den Bildern des Unterkiefers bei vorhandener Referenzierung die benötigte 3D-Darstellung des relevanten Bereichs des Unterkiefers 10 zu erhalten, sofern die Referenzierung 36 den relevanten Bereich des Unterkiefers 10 nicht abschattet.

Mit anderen Worten werden die getrennten Aufnahmen von Unterkiefer mit und ohne Referenzierung durchgeführt, um Fehler, die durch Abschattungen auftreten können, von vornherein zu eliminieren.

Die durch die Referenzierung 36 korrigierten Daten werden sodann vom Rechner 14 derart umgesetzt, dass digitalisierte Daten einer Bearbeitungsmaschine 40 zugeführt werden, mittels der die zahntechnische Restauration hergestellt wird.

Zuvor können dem Rechner 14 gegebenenfalls Daten aus einer Bibliothek 42 zugeführt werden, in der Daten zahntechnischer Restaurationen abgelegt sind.

Ist die erfindungsgemäße Lehre anhand einer zahntechnischen Restauration erläutert worden, so erfolgt hierdurch eine Beschränkung nicht. Vielmehr ist das Verfahren überall dort anwendbar, um von dreidimensionalen Objekten Daten zu gewinnen, die aus Einzelbildern zusammengesetzt sind.

## Patentansprüche

1. Verfahren zur Herstellung einer zahnärztlichen Restauration für zumindest einen Abschnitt eines Zahnbogens (10) auf der Basis von digitalen Gesamtdaten, die aus in Beziehung gesetzten Einzeldaten berechnet werden, denen intraoral durchgeführte dreidimensionale optische Einzelmessungen zumindest eines den Abschnitt enthaltenden Bereichs des Zahnbogens zu Grunde gelegt werden, wobei
der zumindest eine Bereich des Zahnbogens zusammen mit einer dreidimensionale Informationen zur Verfügung stellenden Referenzierung (36, 38) gemessen wird und aus den Einzeldaten unter Berücksichtigung der Referenzierung die Gesamtdaten berechnet werden,
**gekennzeichnet durch** die Verfahrensschritte
a) Durchführen erster intraoraler dreidimensionaler optischer Messungen von dem zumindest einen Bereich des Zahnbogens (10) ohne Vorhandensein der Referenzierung zur Ermittlung erster Einzeldaten,
b) Positionieren der Referenzierung (36, 38) zu dem Bereich,
c) Durchführen zweiter intraoraler dreidimensionaler optischer Messungen von dem Bereich des Zahnbogens oder einem zweiten Bereich, der den Bereich des Zahnbogens umfasst, zur Ermittlung zweiter Einzeldaten und
d) Verknüpfen der ersten Einzeldaten unter Einbeziehung der die Referenzierung berücksichtigenden zweiten Einzeldaten zum Berechnen der Gesamtdaten unter Berücksichtigung von Korrekturdaten aus der Differenz zwischen gemessener und realer Geometrie der Referenzierung
oder
e) Verwenden eines zumindest teilweise transparenten Plattenelements mit in zumindest zwei Ebenen verlaufenden Markierungen als die Referenzierung, Messen des zumindest einen Bereichs des Zahnbogens zusammen mit der Referenzierung und Berechnen der Gesamtdaten.

2. Verfahren nach Anspruch 1,
**dadurch gekennzeichnet,**
**dass** während des Messens des zumindest einen Bereichs des Zahnbogens zusammen mit der Referenzierung (36) diese ortsunveränderlich zu dem Bereich positioniert wird.

3. Verfahren nach Anspruch 1,
**dadurch gekennzeichnet,**
**dass** die Reihenfolge der Verfahrensschritte a) und b), c) ausgetauscht wird.

4. Verfahren nach Anspruch 1,
**dadurch gekennzeichnet,**
**dass** als Referenzierung (36) ein dem Abschnitt körperlich zugeordnetes Objekt verwendet wird, das eine Plattengeorrietrie aufweist.

5. Verfahren nach zumindest Anspruch 4,
**dadurch gekennzeichnet,**
**dass** als Objekt ein solches verwendet wird, von dem geometrische Strukturen wie Pyramiden, Pyramidenstümpfe abragen oder eingelassen sind.

6. Verfahren nach Anspruch 1,
**dadurch gekennzeichnet,**
**dass** die Markierungen auf dem Plattenelement oder in zumindest zwei Ebenen rasterförmig und versetzt zueinander angeordnet werden.

7. Verfahren nach zumindest Anspruch 1,
**dadurch gekennzeichnet,**
**dass** als Referenzierung ein plattenförmiges Element verwendet wird, das aus einem Material niedrigen Ausdehnungskoeffizientens und eines hohen E-Moduls besteht.

8. Verfahren nach zumindest Anspruch 6,
**dadurch gekennzeichnet,**
**dass** als Referenzierung ein plattenförmiges Element verwendet wird, das aus Keramik wie Glaskeramik besteht.

## Claims

1. Method for manufacturing a dental restoration for at least one section of a dental arch (10) on the basis of digital overall data which are calculated on the basis of related individual data, for which are taken as basis three-dimensional optical individual measurements performed on an intra-oral basis of an area that contains at least one section of the dental arch, whereby the at least one area of the dental arch is measured together with a reference (36, 39), which supplies three-dimensional information, and the overall data are calculated from the individual data while considering the reference,
comprising the steps of
a) performing first intra-oral three-dimensional optical measurements of the at least one area of the dental arch (10) without the reference being present to determine first individual data,
b) positioning of the reference (36, 38) in relation to the area,
c) performing second intra-oral three-dimensional optical measurements of the area of the dental arch or a second area, which comprises the area of the dental arch, to determine second individual data and
d) combining the first individual data implicating the second individual data with consideration of the reference to calculate the overall data under consideration of corrective data resulting from the difference between measured and real geometry of the reference
or
e) use of an at least partially transparent plate element with markings that extend on at least two planes as the reference, measurement of the at least one area of the dental arch together with the reference and calculation of the overall data.

2. Method according to claim 1,
**characterized in**
**that** during the measuring of the at least one area of the dental arch together with the reference (36), it is positioned stationary in relation to the area.

3. Method according to claim 1,
**characterized in**
**that** the sequence of the steps a) and b), c) is exchanged.

4. Method according to claim 1,
**characterized in**
**that** as reference (36) is used an object which is physically assigned to the section and has a plate geometry.

5. Method according to claim 4,
**characterized in**
**that** as object is used one from which geometric structures such as pyramids, pyramid stubs protrude or are inserted.

6. Method according to claim 1,
**characterized in**
**that** the markings on the plate element or in at least two planes are positioned in a grid-shaped and offset manner with respect to one another.

7. Method according to claim 1,
**characterized in**
**that** a plate-shaped element is used as reference, which consists of a material with a low expansion coefficient and a high E-modulus.

8. Method according to claim 6,
**characterized in**
**that** a plate-shaped element is used as reference, which is consists of ceramic or glass ceramic.

## Revendications

1. Procédé de fabrication d'une restauration dentaire pour au moins une section d'arcade dentaire (10) sur la base de données numériques générales, qui sont calculées à partir de données individuelles interconnectées, elles-mêmes basées sur des mesures optiques individuelles intra-orales d'au moins une zone contenant la section de l'arcade dentaire,
la zone en question au moins de l'arcade dentaire étant mesurée en même temps qu'un référencement (36, 38) fournissant des informations tridimensionnelles, et les données générales étant calculées à partir des données individuelles avec prise en compte du référencement,
**caractérisé par** les étapes de procédé
a) réalisation de premières mesures optiques tridimensionnelles intra-orales d'au moins une zone de l'arcade dentaire (10) sans présence de référencement pour la détermination de premières données individuelles,
b) positionnement du référencement (36, 38) par rapport à la zone,
c) réalisation de deuxièmes mesures optiques tridimensionnelles intraorales de la zone de l'arcade dentaire ou d'une deuxième zone comprenant la zone de l'arcade dentaire, pour la détermination de deuxièmes données individuelles et
d) connexion des premières données individuelles en intégrant les deuxièmes données individuelles qui tiennent compte du référencement pour le calcul des données générales compte tenu de données correctrices provenant de la différence entre géométrie mesurée et réelle du référencement
ou
e) utilisation d'au moins un élément de plaque partiellement transparent avec des repères pratiqués au moins sur deux niveaux en tant que référencement, mesure d'au moins une zone de l'arcade dentaire en même temps que le référencement pour le calcul des données générales.

2. Procédé suivant la revendication 1,
**caractérisé en ce que**
pendant la mesure d'au moins cette zone de l'arcade dentaire en même temps que le référencement (36) ce dernier est positionné sans déplacement possible par rapport à la zone.

3. Procédé suivant la revendication 1,
**caractérisé en ce que**
l'ordre des étapes de procédé a) et b), c) est inversé.

4. Procédé suivant la revendication 1,
**caractérisé en ce que**
est utilisé comme référencement (36) un objet affecté physiquement à la section et qui présente une géométrie de plaque.

5. Procédé suivant au moins la revendication 4,
**caractérisé en ce que**
est utilisé comme objet un objet dont des structures géométriques telles que pyramides, pyramides tronquées, sont saillantes vers le bas ou noyées.

6. Procédé suivant la revendication 1,
**caractérisé en ce que**
les repères sont agencés sur l'élément de plaque ou en au moins deux niveaux sous forme de grille et sont décalés les uns par rapport aux autres.

7. Procédé suivant au moins la revendication 1,
**caractérisé en ce que**
est utilisé en tant que référencement un élément en forme de plaque composé d'un matériau à faible coefficient de dilatation et à coefficient d'élasticité élevé.

8. Procédé suivant la revendication 6,
**caractérisé en ce que**
est utilisé en tant que référencement un élément en forme de plaque composé d'une céramique telle que la vitrocéramique.
